# EUROPÄISCHE PATENTANMELDUNG

(11) **EP 1 927 411 A2**
(43) Veröffentlichungstag der Anmeldung: **04.06.2008**
(21) Anmeldenummer: 07121758.2
(22) Anmeldetag: 28.11.2007
(51) Int. Cl.: B09C 1/10, E01H 5/00, C12R 1/01

(54) **Mittel zum Abbau von Gefrierpunkt erniedrigenden Mitteln**

(30) Priorität: 29.11.2006 DE 102006056340
(71) Anmelder: Flughafen München GmbH, 85326 München (DE); EMC GmbH, 99086 Erfurt (DE)
(72) Erfinder: Totsche, Kai Uwe, 95463, Bindlach (DE)
(74) Vertreter: Thun, Clemens

(57) **Zusammenfassung**

Die vorliegende Erfindung beschreibt ein Mittel zur Beschleunigung des Abbaus von Gefrierpunkt erniedrigenden Mitteln in Schneedecken, auf befestigten oder unbefestigten Oberflächen oder in Böden, das aus dem Boden isolierte Mikroorganismen umfasst. Die Erfindung betrifft weiterhin ein Verfahren zur Beschleunigung des Abbaus von Gefrierpunkt erniedrigenden Mitteln unter Verwendung des Mittels.

## Beschreibung

Die vorliegende Erfindung betrifft ein Mittel zum Abbau von Gefrierpunkt erniedrigenden Mitteln, wie beispielsweise Enteisungsmittel in Böden oder Bodennähe sowie ein Verfahren zum Abbau von Gefrierpunkt erniedrigenden Mitteln im Boden oder in Bodennähe unter Verwendung dieses Mittels.

Auf den Betriebsgeländen von Flughäfen werden im Winter hohe Mengen an Enteisungsmitteln eingesetzt, um Flugzeuge, Vorfelder, Rollwege und Startbahnen von Schnee und Eis zu befreien oder von Schnee und Eis frei zu halten, um auf diese Weise die erforderliche Verkehrssicherheit zu gewährleisten. Auf dem Betriebsgelände eines großen Verkehrsflughafens werden in der Regel mehrere Tausend Tonnen Enteisungsmittel jährlich aufgewendet. In der Regel kommen hier Stoffe bzw. Stoffgemische auf der Grundlage organischer Verbindungen, wie beispielsweise Acetate, Formiate und Glykole, zum Einsatz.

Ein erheblicher Anteil dieser Enteisungsmittel wird von den behandelten Flächen und Flugzeugen durch Wind oder Triebwerkstrahl abgeschert und anschließend verblasen, wodurch sie in die Umgebung der Enteisungsmittel-Einsatzorte gelangen und sich dort diffus auf der Geländeoberfläche niederschlagen. Dann infiltrieren sie entweder direkt in den Boden oder sie akkumulieren zunächst in der Schneeauflage oder auf der gefrorenen Oberfläche, bevor sie in den Boden infiltrieren. Insgesamt kommt es zu einer beträchtlichen Kontamination der Böden durch die Enteisungsmittel. Bei geeigneten Wetterlagen (Schneeschmelze) können große schubweise Belastungen auftreten.

Grundsätzlich sind Enteisungsmittel aerob und anaerob abbaubar. In begrenztem Maß können die Enteisungsmittel auch in Böden abgebaut werden. Das natürliche Abbauvermögen der Böden für diffuse Enteisungsmittel-Einträge ist allerdings durch verschiedene Faktoren limitiert. Dieses ist insbesondere der Fall bei geringen Aufenthaltszeiten des Sickerwassers im Boden. Folgende Ereignisse sind dabei relevant:
(i) bei einer Schneeschmelze, wenn hohe Sickerwassermengen anfallen und damit zugleich hohe Sickergeschwindigkeiten vorherrschen;
(ii) bei punktueller Infiltration höherer Mengen Enteisungsmittel, die aufgrund der räumlichen Variabilität von Bodenparametern, aufgrund selbstverstärkender Effekte durch die Herabsetzung der Oberflächenspannung des Enteisungsmittel-WasserGemisches oder an ungefrorenen Bereichen im sonst gefrorenen Boden auftreten kann;
(iii) bei ungünstigen Bodeneigenschaften oder Bodenaufbau, beispielsweise geringe Mächtigkeit des Oberbodens, hohe ungesättigte Wasserleitfähigkeit und geringer Grundwasser-Flurabstand und
(iv) bei Abwesenheit von spezifischen Mikroorganismen.

Wenn kein vollständiger Abbau in oberflächennahen Bodenschichten stattfindet, gelangen die Enteisungsmittel in größere Bodentiefen und nachfolgend in das Grundwasser. Aufgrund der geringen Nachlieferung von Luftsauerstoff an diesen Orten etablieren sich anaerobe Verhältnisse. Für den weiteren Abbau fungieren dann als Endelektronenakzeptoren Nitrat, Eisen, Mangan und Sulfat. Außerdem steigt die Gefahr der Methanogenese, je anaerober das Milieu wird. Die Bildung von Methan ist aber sowohl aus Gründen des Umweltschutzes als auch aus Gründen der Betriebssicherheit unbedingt zu vermeiden.

Aufgrund nationaler und europäischer Gesetze ist sowohl der Eintrag von Fremdstoffen (und somit auch der Eintrag von Enteisungsmittel) in das Grundwasser als auch die Veränderung der Beschaffenheit des Grundwassers (also auch die mit dem Abbau von Enteisungsmittel einhergehenden Änderungen in der stofflichen Zusammensetzung des Grundwassers) zu verhindern oder zumindest auf ein verträgliches Maß zu beschränken. Bezüglich des Bodens muss vor dem Hintergrund der nationalen Gesetze der mit dem Eintrag von Enteisungsmittel verbundene Eingriff auf ein Maß beschränkt werden, das keine erheblichen Beeinträchtigungen der Bodenfunktionen nach sich zieht.

Es ist daher Aufgabe der vorliegenden Erfindung, ein spezifisches umweltfreundliches Mittel anzugeben, das den Abbau von Gefrierpunkt erniedrigenden Mittel, wie z. B. Enteisungsmitteln in Schneedecken, auf befestigten und unbefestigten Flächen und in Böden fördert und dadurch das Eindringen von Enteisungsmittel in größere Bodentiefen und/oder in das Grundwasser weitgehend unterbindet.

Es ist eine weitere Aufgabe der vorliegenden Erfindung, ein Verfahren zur Verfugung zu stellen, mit dem der Einsatz dieses Mittels sorgfältig geplant und kontrolliert werden kann, mit dem Ziel, durch eine umweltschonende Technologie die natürliche Bodenfunktionen zu erhalten und Gefahren einer erheblichen Kontamination des Grundwassers mit diesen Mitteln zu vermeiden.

Diese Aufgaben werden mit dem Mittel und mit dem Verfahren der vorliegenden Erfindung gelöst.

Die vorliegende Erfindung betrifft ein Mittel zur Beschleunigung des Abbaus von Gefrierpunkt erniedrigenden Mitteln, wie Enteisungsmitteln in Böden oder Bodennähe, das spezielle aus dem Boden isolierte Mikroorganismen beinhaltet.

Die Erfindung betrifft weiterhin ein Verfahren zur Beschleunigung des Abbaus von Gefrierpunkt erniedrigenden Mitteln, wie Enteisungsmitteln, im Boden und in Bodennähe, z. B. in Schneedecken, auf befestigten und unbefestigten Flächen, wobei das erfindungsgemäße Mittel auf mit Gefrierpunkt erniedrigenden Mitteln, wie Enteisungsmittel, belastete Oberflächen (z. B. Grünland, Schneedecke, befestigte Flächen) aufgetragen wird.

Die Unteransprüche definieren bevorzugte Ausführungsformen des erfindungsgemäßen Mittels sowie des erfindungsgemäßen Verfahrens.

Die vorliegende Erfindung betrifft ebenfalls die Verwendung des erfindungsgemäßen Mittels auf Böden und in Bodennähe, die mit Gefrierpunkt erniedrigenden Mitteln, wie Enteisungsmitteln, kontaminiert sind.

Das erfindungsgemäße Mittel ist in der Lage, spezifisch den Abbau von Gefrierpunkt erniedrigenden Mitteln, wie Enteisungsmitteln, in Schneedecken, auf befestigten und unbefestigten Flächen und in Böden zu ermöglichen bzw. zu beschleunigen.

Das erfindungsgemäße Mittel enthält spezifische, aus dem Boden isolierte, an den Abbau von Enteisungsmitteln unter niedrigen Temperaturen adaptierte Mikroorganismen. In einer bevorzugten Ausführungsform der vorliegenden Erfindung sind die in dem Mittel enthaltenen Mikroorganismen standortspezifisch.

Es ist zu berücksichtigen, dass durch den Einsatz des Mittels ein bestimmtes Maß an biologischer Aktivität, d. h. eine bestimmte Menge an vitalen Mikroorganismen, auf den mit dem Mittel behandelten Flächen zu erzielen ist. In der Praxis hat sich herausgestellt, dass bei einer Applikationsmenge von 0,05 - 0,2 l / m² die Mikroorganismen eine Konzentration zwischen 10⁵ und 10¹² KBE/ml aufweisen sollten. Die bevorzugte Konzentration liegt bei > 10⁸ KBE/ml.

Die Mikroorganismen, die in dem erfindungsgemäßen Mittel enthalten sind, sind spezifische, bodenbürtige, an den Abbau von Gefrierpunkt erniedrigenden Mitteln, wie Enteisungsmitteln, unter niedrigen Temperaturen adaptierte Mikroorganismen. Bevorzugte Mikroorganismen sind Bakterien beispielsweise die Gattungen Azospirillum, Azidovorax, Rhodococcus, Pseudoxanthomonas, Xanthomonas und Pseudomonas sowie Mischungen daraus. Besonders bevorzugt sind die Arten Azidovorax sp., Azospirillum lipoferum, Rhodococcus erythropolis, Pseudomonas putida, Pseudomonas brenneri, Pseudomonas rhodesia, Pseudomonas veronii, Pseudomonas marginalis, Pseudoxanthomonas mexicana und Mischungen daraus.

Die Mikroorganismen können wie folgt durch ein Selektionsverfahren ausgewählt werden. Zunächst wird ein ausgewähltes Bodenmaterial, zum Beispiel ein solches, das mit Gefrierpunkt erniedrigenden Mitteln, wie Enteisungsmitteln belastet ist, mit einer wässrigen Lösung perkoliert, die die Enteisungsmittel enthält, die durch die Mikroorganismen abgebaut werden. Dadurch erhält man ein Sickerwasser, das ein Konsortium von bodenbürtigen Mikroorganismen enthält. Das Sickerwasser wird von Spezies dominiert, die das Enteisungsmittel als Nahrungsquelle nutzen können, also zu einem Abbau desselben befähigt sind. Die Perkolation erfolgt unter Bedingungen, die an den späteren Einsatzzweck angepasst sind, z. B. niedrige Temperaturen.

Die wässrige Perkolationslösung kann neben dem Enteisungsmittel in Abhängigkeit von beispielsweise der Natur des Enteisungsmittels und der späteren Verwendung der zu selektierenden Mikroorganismen noch weitere Additive enthalten. Als Additive können beispielsweise Nährstoffe, Co-Substrate, Vitamine, Mobilitätsvermittler, Spurenelemente und Sauerstoffdonatoren in solchen Mengen zugegeben werden, die einen vollständigen Abbau ermöglichen und/oder einen bestimmten Abbauweg fördern.

Diese Additive dienen dazu, bestimmte Interaktionen zwischen dem Sickerwasser und dem abiotischen Bestandteilen des Bodens zu fordern beziehungsweise zu unterdrücken und/oder um bestimmte chemische Lebensbedingungen im Sickerwasser für die Mikroorganismen einzustellen.

Es kann auch mehr als einmal perkoliert werden, d. h., die Perkolation wird wiederholt durchgeführt in Abhängigkeit des Bodens, der zu isolierenden Mirkoorganismen und weiteren Parametern.

Zielstellung für die Perkolation des Bodenmaterials ist es, dass sich im Sickerwasser möglichst ausschließlich solche Mikroorganismen anreichern, die zum Abbau der organischen Substanz unter den vorgegebenen Bedingungen befähigt sind. Dieses Ziel wird durch eine intermittierende Perkolation des Bodenmaterials erreicht. In den Ruhephasen zwischen zwei Perkolationsphasen findet eine In-Situ-Inkubation des Sickerwassers im Bodenmaterial statt. Während dieser In-Situ-Inkubation werden die Mikroorganismen selektiert und angereichert, die zum Abbau der Enteisungsmittel befähigt sind. Bei der anschließenden Perkolationsphase werden diese selektierten und angereicherten Mikroorganismen mit dem Sickerwasser ausgewaschen.

Die Perkolation des Bodens zur Gewinnung des Sickerwassers kann sowohl im Freiland als auch im Labor durchgeführt werden. Bei der Sickerwassergewinnung im Freiland werden bevorzugt Lysimeter, Saugkerzen, Saugplatten oder Drainagen verwendet. Bei der Durchführung der Perkolation im Labor hat sich eine Durchführung in Lysimetern oder Bodensäulen als praktikabel erwiesen.

Die Temperatur während der Perkolation ist in Abhängigkeit der zu isolierenden Mikroorganismen und der späteren Verwendung derselben festzulegen. Normalerweise wird die Perkolation bei einer Temperatur im Bereich von 0 °C bis 40 °C durchgeführt.

Nach der Perkolation wird das Sickerwasser fraktioniert, in geeigneten Behältern gesammelt und für die Inkubationsversuche nach üblichen Verfahren homogenisiert.

Nach der Gewinnung des Sickerwassers werden mit diesem Inkubationsversuche unter bestimmten chemischen und physikalischen Umweltbedingungen durchgeführt. Dies umfasst die Einstellung eines geeigneten pH-Wertes, einer geeigneten Temperatur, einer geeigneten Ionenstärke sowie einer geeigneten Zusammensetzung der Lösung. Dadurch werden die Spezies selektiert, die das Gefrierpunkt erniedrigende Mittel, wie Enteisungsmittel, unter den gegebenen Bedingungen am besten abbauen können.

Die Inkubation kann in wässriger Phase erfolgen oder sie kann auch in einem System aus wässriger Phase und Festphase durchgeführt werden.

Bei den Inkubationsversuchen sind die einzustellenden chemischen und physikalischen Umweltbedingungen aufgabenspezifisch festzulegen. Insbesondere ist hier maßgeblich, unter welchen Bedingungen die Mikroorganismen später zur Anwendung gelangen sollen.

Im Gegensatz zur Perkolation verfügen die Mikroorganismen nicht mehr über ihren natürlichen Lebensraum, d. h. den Boden. In Abhängigkeit des Einsatzgebietes der Mikroorganismen besteht der künstliche Lebensraum bei den Inkubationsversuchen nur aus einer wässrigen Phase oder aus einer Kombination aus wässriger und (künstlicher) Festphase, zum Beispiel ein Festbettreaktor.

Während der Inkubation findet ein starkes Wachstum der Mikroorganismen statt. Während dieses Wachstumsprozesses werden die Spezies verdrängt, die die Enteisungsmittel im künstlichen Lebensraum unter den gegebenen chemischen und physikalischen Bedingungen nicht oder nur schlecht als Nahrungsquelle nutzen können. Man erhält auf diese Weise ein Konsortium an Mikroorganismen, das Enteisungsmittel unter den gegebenen Bedingungen außerhalb ihrer natürlichen Umgebung abzubauen vermag.

Zur Einstellung dieser chemischen und physikalischen Umweltbedingungen gehört beispielsweise dazu, dass die Inkubation des Sickerwassers in Gegenwart des Enteisungsmittels durchgeführt wird. Vorzugsweise werden ein Sauerstoffdonator, Wachstumsnährstoffe und/oder Suppine hinzugefügt.

Ein bevorzugter Sauerstoffdonator ist beispielsweise eine Nitratverbindung. Zu den Wachstumsnährstoffen zählen beispielsweise Quellen für elementare Nährstoffe, wie Stickstoff, Phosphor, Kalium und Schwefel. Des Weiteren können Suppine zugeführt werden, zu denen beispielsweise Vitamine und Spurenelemente zählen. Die Vitamine werden entweder in Reinform oder in Form von Trockenhefe, Hefeextrakt oder Bierhefe eingesetzt.

Während der Inkubation werden physiko-chemische Parameter gemessen. Beispiele für diese physiko-chemischen Parameter sind der pH-Wert, die elektrische Leitfähigkeit, der DOC-Wert, die Trübung, der Sauerstoffgehalt, der Kohlendioxidgehalt, der Gehalt an organischen Substanzen und deren Metabolite sowie Anionen und Kationen. Die Inkubationsversuche werden durchgeführt, bis kein weiteres Wachstum der Mikroorganismen mehr zu beobachten ist.

Anschließend erfolgt die Identifizierung der die organische Substanz abbauenden Mikroorganismen. Die Identifikation der Mikroorganismen erfolgt beispielsweise mit klassischen gentechnischen Verfahren, wobei die ribosomale RNS mittels Polymerase-Ketten-Reaktion amplifiziert und ansschließend sequenziert und mit Gen-Datenbanken verglichen wird.

Das erfindungsgemäße Mittel enthält bevorzugt einen Sauerstoffdonator. Geeignete Sauerstoffdonatoren sind stickstoffhaltige Verbindungen, wie beispielsweise ein Nitrat. Weitere mögliche Sauerstoffdonatoren sind Sulfate, Eisen(III)-oxihydroxide oder Mangan(IV)-oxide. Auch H₂-Gas stellt einen möglichen Endelektronenakzeptor dar.

In einer weiteren bevorzugten Ausführungsform der vorliegenden Erfindung enthält das Mittel weiterhin Wachstumsnährstoffe. Dazu gehören Quellen für elementare Nährstoffe, wie Stickstoff, Phosphor, Kalium und Schwefel. Des Weiteren kann das erfindungsgemäße Mittel Suppine enthalten, zu denen beispielsweise Vitamine und Spurenelemente zählen.

Die Menge des in dem Mittel enthaltenen Sauerstoffdonators richtet sich nach der Einsatzmenge des Enteisungsmittels, das mit dem Mittel abzubauen ist und/oder nach den gemessenen Immissionen an der Boden- bzw. Schneeoberfläche unter Beachtung der meteorologischen Bedingungen. Die Konzentration des Sauerstoffdonators liegt typischerweise zwischen 1 mol/l und 10 mol/l.

Zur Verringerung der Materialkosten können der Sauerstoffdonator und die Wachstumsnährstoffe in Form von geeigneten landwirtschaftlichen Düngemitteln eingesetzt werden. Ein Beispiel dafür ist Nitrophoska. An Stelle der Vitamine in Reinform können auch Trockenhefe oder Hefeextrakt enthalten sein.

In einer weiteren bevorzugten Ausführungsform der vorliegenden Erfindung liegt das Mittel als wässrige Lösung vor. Alternativ kann es auch als streufähiges Pulver konfektioniert werden.

In einer bevorzugten Ausführungsform ist das Enteisungsmittel eine organische Verbindung. In einer bevorzugten Ausführungsform ist das Enteisungsmittel eine Glykolverbindung, beispielsweise Propylenglykol, ein Acetat, ein Formiat, beispielsweise Kaliumformiat oder eine Mischung daraus.

Das erfindungsgemäße Mittel weist die folgenden Vorteile auf: Die darin enthaltenen Mikroorganismen sind natürlicherweise am Standort vorhanden, sodass keine wasserrechtlichen Bedenken zu erwarten sind. Durch die Zugabe von Wachstumsnährstoffen und des Sauerstoffdonators werden ein rasches Wachstum und damit ein schneller Abbau der Enteisungsmittel im Boden erreicht. Die Mikroorganismen sind an niedrige Umgebungstemperaturen und sogar an Gefriertemperaturen adaptiert und bauen auch unter Winterbedingungen die Enteisungsmittel ab. Sofern das Mittel gezielt auf unbefestigten Flächen eingesetzt wird, gelangen bei der Sammlung der Enteisungsabwässer von befestigten Flächen keine Mikroorganismen in mögliche nachgeschaltete Anlagen zum Recycling der Enteisungsmittel.

Die Immission von Enteisungsmittel auf unbefestigte Flächen insbesondere an Flughäfen ist systembedingt nahezu unvermeidbar. Bislang wird einzig durch großtechnische Lösungen, die gänzlich auf den Einsatz von chemischen Enteisungsmitteln verzichten, wie beispielsweise die Beheizung von Rollwegen, die Flugenteisung durch Einsatz von Mikrowellen, versucht, die beschriebene Problematik zu minimieren.

Grundsätzlich kann das erfindungsgemäße Mittel auf allen Flächen, die mit Gefrierpunkt erniedrigenden Mitteln, wie Enteisungsmitteln, kontaminiert sind, verwendet werden. Es kann sich um befestigte Flächen oder unbefestigte Flächen sowie Böden handeln. Die zu behandelnden Flächen können eine Auflage aus Schnee oder Eis aufweisen. Solche Flächen finden sich beispielsweise in der Umgebung von Flughäfen, Autobahnen und Schienenwegen.

Die vorliegende Erfindung stellt ebenfalls ein Verfahren zur Beschleunigung des Abbaus von Enteisungsmittel in Böden oder in Bodennähe, wie in Schneedecken, auf befestigten und unbefestigten Flächen, zur Verfügung, wobei das erfindungsgemäße Mittel auf mit Enteisungsmitteln belastete Oberflächen aufgetragen wird.

In einer bevorzugten Ausführungsform des erfindungsgemäßen Verfahrens wird das Mittel in Form einer wässrigen Lösung eingesetzt. Diese wässrige Lösung enthält bevorzugt einen Sauerstoffdonator sowie Wachstumsnährstoffe und/oder Suppine, die bereits zuvor im Einzelnen erläutert worden sind.

Die Ausbringung des Mittels kann mit üblichem landwirtschaftlichen Gerät, wie beispielsweise einem Spritzgerät, erfolgen. Die Entscheidung, zu welchen Zeitpunkten das Mittel anzuwenden ist, erfolgt in Abhängigkeit von den zuvor eingesetzten Mengen an Enteisungsmitteln und/oder von gemessenen Immissionen an der Flächenbeziehungsweise Schneeoberfläche unter Beachtung der meteorologischen Randbedingungen. Die Dosierung des Mittels bei der Anwendung hängt von den Mikroorganismen ab und erfolgt unter der Maßgabe, durch die Ausbringung ein bestimmtes Maß an biologischer Aktivität auf der Applikationsfläche zu erzielen. Die Spanne der geeigneten Dosierungen reicht von 0,01 l/m² bis 10 l/m². Typisch sind Dosierungen zwischen 0,05 l/m² und 0,2 l/m².

Zur Herstellung des erfindungsgemäßen Mittels zur Beschleunigung des Abbaus von Enteisungsmitteln werden die Mikroorganismen in geeigneter Weise kultiviert und zur vorübergehenden Lagerung des Mittels konzentriert und/oder konserviert. Vor der Anwendung ist eine Reaktivierung des Abbaubeschleunigers sowie eine Zugabe von einem Sauerstoffdonator und gegebenenfalls weiteren Wachstumsnährstoffen und Suppinen durchzuführen.

Wie bereits eingangs beschrieben wurde, enthält das erfindungsgemäße Mittel spezifische, aus dem Boden isolierte (bodenbürtige) Mikroorganismen, die auf mit Enteisungsmittel kontaminierten beziehungsweise belasteten Flächen aufgebracht werden. Es kommen Mikroorganismen zum Einsatz, die bevorzugt natürlicher Weise am Standort bereits vorhanden sind, die sich möglicherweise über Jahre hinweg an den Abbau von Enteisungsmittel angepasst haben.

Zur Herstellung des erfindungsgemäßen Mittels ist zunächst die Vermehrung der aus dem Boden isolierten und selektierten Mikroorganismen erforderlich. Dazu werden die Mikroorganismen in einem wässrigen Medium, das Enteisungsmittel, einen Sauerstoffdonator, Wachstumsnährstoffe und Suppine enthält, kultiviert. Diese Kultivierung der Mikroorganismen erfolgt in geeigneten Fermentoren. Die Umgebungsbedingungen (Temperatur) sind auf den späteren Einsatzzweck abgestimmt. Dem Kultivierungsmedium zur Anreicherung der Mikroorganismen wird das Enteisungsmittel als Kohlenstoffquelle zugesetzt. Weiterhin werden dem Kultivierungsmedium der Sauerstoffdonator, zum Beispiel ein Nitrat, weitere elementare Nährstoffe, beispielsweise Stickstoff, Phosphor, Kalium, Schwefel, sowie Suppine, wie Vitamine, Spurenelemente, zugegeben. Die Zugabe der Kohlenstoffquelle erfolgt derart, dass einerseits ein optimales Wachstum der Mikroorganismen erzielt wird, andererseits aber auch ein vollständiger Abbau des Enteisungsmittel während der Kultivierung erfolgt. Der Sauerstoffdonator wird in der Weise dosiert, dass er den zum Abbau des eingesetzten Enteisungsmittel erforderlichen Sauerstoffbedarf decken kann.

Nach der Kultivierung der Mikroorganismen werden diese konzentriert und/oder konserviert. Die Konzentrierung kann beispielsweise durch Cross-Flow-Filtration erfolgen. Für die Konservierung werden übliche Verfahren eingesetzt, wozu beispielsweise das Gefrieren, die Gefriertrocknung, die Wirbelschichttrockung sowie die Immobilisierung auf Schaumstoffträger zählen.

Zur Vorbereitung der Anwendung des Mittels wird eine Reaktivierung des Mikroorganismenkonzentrats beziehungsweise -konservats durchgeführt. Dazu wird das Konzentrat/Konservat in einer wässrigen Lösung resuspendiert, die das Enteisungsmittel, einen Sauerstoffdonator, Wachstumsnährstoffe und Suppine, wie beispielsweise Vitamine, enthält. Die Menge an Sauerstoffdonator hängt von der auf den Applikationsflächen mit dem Mittel abzubauenden Menge an Enteisungsmitteln ab. Die weiteren Additive werden so eingesetzt, dass sich in der gebrauchsfertigen Lösung die gleichen Konzentrationen wie bei der Kultivierung der Mikroorganismen ergeben.

Die Standzeit des Reaktivats vor der Ausbringung sowie das Temperaturregime während der Reaktivierung und der anschließenden Standzeit werden auf das Konzentrations- und Konservierungsverfahren sowie auf die Rahmenbedingungen für die Anwendung abgestimmt.

Das folgende Beispiel dient zur Erläuterung der vorliegenden Erfindung.

### BEISPIEL

### 1. Kultivierung der Mikroorganismen und Konservierung

Zur Kultivierung wurden die Mikroorganismen Azospirillum lipoferum und Acidovorax sp. verwendet. Die Kultivierung erfolgte als fed-batch-Fermentation und Konti-Hochzelldichte-Fermentation über mehrere Fermentorstufen, bis eine Zellzahl von mindestens 10⁸ KBE (Kolonie bildende Einheiten) erreicht wurde. Anschließend wurde die Kultivierungslösung mittels Cross-Flow-Filtration auf eine Zellzahl von mindestens 10¹⁰ KBE konzentriert und das Konzentrat zur Konservierung in 10 l-Portionen eingefroren. Die Kultivierungslösung wies folgende Zusammensetzung auf:
➢ Enteisungsmittel: 2 g/l Typ I Safewing MP I 1938 Aircraft De-icer, 0,5 g/l Typ IV Safewing MP IV 2001 Aircraft De-icer (Wirksubstanz: Propylenglykol)
➢ Sauerstoffdonator: 17,4 g/l NH₄NO₃
➢ Basalmedium: 0,62 g/l (NH₄)₂SO₄, 3,50 g/l K₂HPO₄,
➢ Vitamine: 0,5 g/l autolysierte Bierhefe (BH)
➢ Spurenelemente: 1 ml/l Spurenelementlösung
➢ pH 7 (Einstellung mit NaOH)

### Zusammensetzung der Spurenelement-Lösung

| **Komponente** | **g/l Medium** |
|---|---|
| FeCl₂ x 4 H₂O | 1,500 |
| CoCl₂ x 6 H₂O | 0,190 |
| NiCl₂ x 6 H₂O | 0,024 |
| CuCl₂ x 2 H₂O | 0,002 |
| MnCl₂ x 4 H₂O | 0,100 |
| ZnCl₂ | 0,070 |
| H₃Bo₃ | 0,006 |
| Na₂MoO₄ x 2 H₂O | 0,036 |

### 2. Reaktivierung des Mittels

Die Reaktivierung wurde in einer ungeheizten Halle durchgeführt. Zur Reaktivierung wurden 100 l des Konzentrats in 5.000 l Wasser (Temperatur 30 °C) resuspendiert. Die Resuspendierungslösung enthielt 2 kg Enteisungsmittel (80 % Typ 1, 20 % Typ4, siehe oben), 132,5 kg Kalkammonsalpeter als Sauerstoffdonator, 47,5 kg Nitrophoska 15-15-15 (+2S) als Basalmedium, 5 l Spurenelementlösung und 0,25 kg Hefeextrakt. Das Reaktivat wurde unter ständigem Umpumpen (mit einer Pumprate von 5 m³/h) für 48 h einer Nachreifung unterzogen. Während dieses Nachreifungsprozesses kühlte das Reaktivat nahezu auf Raumtemperatur (zwischen 4 °C und 10 °C, abhängig von der Außentemperatur) ab.

### 3. Ausbringen des Mittels

Der gebrauchsfertiger Abbaubeschleuniger wurde mittels eines landwirtschaftlichen Spritzgerätes (Spritzbreite 30 m) auf eine Fläche von 50.000 m² in einer Dosis von 0,1 l/m² ausgebracht.

Bei höheren Immissionen kann vor der Applikation das Reaktivat mit 5.000 l Wasser verdünnt werden, das ebenfalls Raumtemperatur aufwies und 415 kg Kalkammonsalpeter enthielt.

Die Soll-Termine der Reaktivierung und Applikation wurden derart ermittelt, dass im Zeitraum zwischen 2 Sollterminen auf den der Applikationsfläche zuzuordnenden Emissionsflächen jeweils 1/30 der durchschnittlichen jährlichen Gesamtmenge an Enteisungsmittel eingesetzt wurden. Wenn zum Soll-Termin der Applikation meteorologische Ausschlusskriterien vorlagen oder die Applikation aus technischen Gründen nicht möglich war, wurde die Applikation zum nächstmöglichen Zeitpunkt nachgeholt. Bei Sonderereignissen (z. B. Schneeschmelze) wurde der Soll-Termin zur Optimierung des Behandlungserfolges ggf. um einige Tage früher oder später durchgeführt.

## Patentansprüche

1. Mittel zur Beschleunigung des Abbaus von Gefrierpunkt erniedrigenden Mitteln in Böden oder Bodennähe, das aus dem Boden isolierte Mikroorganismen umfasst.

2. Mittel nach Anspruch 1,
**dadurch gekennzeichnet,**
**dass** die Mikroorganismen standortspezifisch sind.

3. Mittel nach Anspruch 1 oder 2,
**dadurch gekennzeichnet,**
**dass** es einen Sauerstoffdonator bzw. Elektronenakzeptator enthält.

4. Mittel nach Anspruch 3,
**dadurch gekennzeichnet,**
**dass** der Sauerstoffdonator eine stickstoffhaltige Verbindung ist.

5. Mittel nach Anspruch 4,
**dadurch gekennzeichnet,**
**dass** die stickstoffhaltige Verbindung eine Nitrat-Verbindung ist.

6. Mittel nach mindestens einem der Ansprüche 1 bis 5,
**dadurch gekennzeichnet**,
**dadurch gekennzeichnet, dass** es Wachstumsnährstoffe enthält.

7. Mittel nach Anspruch 6,
**dadurch gekennzeichnet,**
**dass** es Suppine enthält.

8. Mittel nach mindestens einem der Ansprüche 1 bis 7,
**dadurch gekennzeichnet,**
**dass** es als wässrige Lösung vorliegt.

9. Mittel nach mindestens einem der Ansprüche 1 bis 8,
**dadurch gekennzeichnet,**
**dass** die Mikroorganismen in einer Konzentration von 10⁵ bis 10¹² KBE/100 ml vorhanden sind.

10. Mittel nach mindestens einem der Ansprüche 1 bis 9,
**dadurch gekennzeichnet,**
**dass** die Mikroorganismen Bakterien sind.

11. Mittel nach Anspruch 10,
**dadurch gekennzeichnet,**
**dass** die Mikroorganismen aus Azidovorax, Azospirillum, Rhodococcus, Pseudomonas, Pseudoxanthomonas, Xanthomonas und Mischungen daraus gewählt sind.

12. Mittel nach Anspruch 11,
**dadurch gekennzeichnet,**
**dass** die Mikroorganismen aus Azidovorax sp., Azospirillum lipoferum, Rhodococcus erythropolis, Pseudomonas putida, Pseudomonas brenneri, Pseudomonas rhodesia, Pseudomonas veronii, Pseudomonas marginalis, Pseudoxanthomonas mexicana und Mischungen daraus gewählt sind.

13. Mittel nach mindestens einem der Ansprüche 1 bis 12,
**dadurch gekennzeichnet,**
**dass** das Gefrierpunkt erniedrigende Mittel ein Enteisungsmittel ist, das aus einer Glykolverbindung, einem Acetat, einem Formiat oder einer Mischung daraus gewählt ist.

14. Verfahren zur Beschleunigung des Abbaus von Gefrierpunkt erniedrigenden Mitteln im Boden oder in Bodennähe,
**gekennzeichnet durch**
Austragen eines Mittels nach mindestens einem der Ansprüche 1 bis 13 auf mit Gefrierpunkt erniedrigenden Mitteln belastete Flächen.

15. Verfahren nach Anspruch 14,
**dadurch gekennzeichnet,**
**dass** das Mittel in Form einer wässrigen Lösung eingesetzt wird.

16. Verfahren nach Anspruch 14 oder 15,
**dadurch gekennzeichnet,**
**dass** das Mittel in einer Menge von 0,01 bis 10 l/m² ausgetragen wird.

17. Verfahren nach Anspruch 16,
**dadurch gekennzeichnet,**
**dass** das Mittel mit einem landwirtschaftlichen Gerät ausgetragen wird.

18. Verfahren nach mindestens einem der Ansprüche 14 bis 17,
**dadurch gekennzeichnet,**
**dass** das Mittel auf die Schneedecke aufgetragen wird.

19. Verfahren nach mindestens einem der Ansprüche 14 bis 17,
**dadurch gekennzeichnet,**
**dass** das Mittel auf befestigte oder unbefestigte Oberflächen aufgetragen wird.

20. Verfahren zur Herstellung eines Mittels zur Beschleunigung des Abbaus von Gefrierpunkt erniedrigenden Mitteln nach mindestens einem der Ansprüche 1 bis 13, mit den Schritten:
Kultivierung der Mikroorganismen in einem wässrigen Medium, das
Gefrierpunkt erniedrigendes Mittel, einen Sauerstoffdonator,
Wachstumsnährstoffe und Suppine enthält und
- Konzentrierung und/oder Konservierung der kultivierten
Mikroorganismen.

21. Verfahren nach Anspruch 20,
**dadurch gekennzeichnet,**
**dass** das Mikroorganismenkonzentrat bzw. -konservat reaktiviert wird.

22. Verfahren nach Anspruch 21,
**dadurch gekennzeichnet,**
**dass** die Reaktivierung in einer wässrigen Lösung durchgeführt wird, die das Gefrierpunkt erniedrigende Mittel, einen Sauerstoffdonator, Wachstumsnährstoffe und Suppine enthält.

23. Verwendung eines Mittels nach mindestens einem der Ansprüche 1 bis 13 auf Böden und/oder bodennahen Flächen, die mit Gefrierpunkt erniedrigenden Mitteln kontaminiert sind.

24. Verwendung nach Anspruch 22,
**dadurch gekennzeichnet,**
**dass** sich diese Böden an Flughäfen, Autobahnen und Schienenwegen befinden.
